# EUROPEAN PATENT SPECIFICATION

(11) **EP 3 919 121 B1**
(45) Date of publication and mention of the grant of the patent: **13.08.2025**
(21) Application number: 20748930.3
(22) Date of filing: 31.01.2020
(51) Int. Cl.: A61N 5/06, A61B 5/00

(54) **PHOTOTHERAPY APPARATUS**
LICHTTHERAPIEVORRICHTUNG
APPAREIL DE PHOTOTHÉRAPIE

(30) Priority: 31.01.2019 US 201962799728 P
(43) Date of publication of application: 08.12.2021
(73) Proprietor: Seoul Viosys Co., Ltd., Ansan-si, Gyeonggi-do 15429 (KR)
(72) Inventor: YOON, Yeong Min, Ansan-Si Gyeonggi-do 15429 (KR); BAE, Hee Ho, Ansan-Si Gyeonggi-do 15429 (KR); LEE, A Young, Ansan-Si Gyeonggi-do 15429 (KR)
(74) Representative: Stolmár & Partner Patentanwälte PartG mbB
(86) International application number: PCT/KR2020/001481
(87) International publication number: WO 2020/159280

(56) References cited:
- JP-A- 2000 217 939
- JP-A- 2002 000 745
- KR-A- 20160 070 930
- KR-A- 20160 070 930
- KR-A- 20160 147 585
- KR-A- 20160 147 585
- KR-B1- 100 787 874
- US-A1- 2001 053 907
- US-A1- 2013 041 308
- US-A1- 2016 129 279
- US-A1- 2018 117 355
- US-A1- 2018 289 421

## Description

### [Technical Field]

Embodiments of the present disclosure relate to a phototherapy apparatus.

### [Background Art]

Light has different properties depending on wavelength thereof. Recently, apparatuses using various wavelengths of light have been developed and put into use.

Particularly, UV light capable of killing cells of an organism through destruction of DNA is used in therapeutic devices for treatment of infections through destruction of bacteria. However, a conventional therapeutic device using UV light has a problem in that, upon treatment of an infected site, UV light is delivered not only to the infected site but also to a normal body site.

In addition, an infrared light-based therapeutic device, which heats an infectious agent to a temperature high enough to cause death of the infectious agent, has a problem in that a subject to be treated can feel pain due to intense heat.

US 2001/053907 A1 discloses a laser treatment apparatus for irradiating a skin with a laser beam for treatment, comprising a treatment part detection unit, a a distance detection unit, and a movement unit which moves the emission end unit with respect to the treatment part.

US 2018/289421 A1 discloses a system for treating a dermal fungal infection in a mammal, the system comprising: a laser; an imaging unit for obtaining infection image data; and an alignment stage for receiving an infected portion of a mammal comprising the dermal fungal infection and aligning the infected portion of the mammal with the laser.

### [Disclosure]

### [Technical Problem]

Embodiments of the present disclosure provide a phototherapy apparatus which ensures exact delivery of therapeutic light to a treatment site and thus can prevent other normal sites from being damaged by the therapeutic light.

### [Technical Solution]

In accordance with an aspect of the present disclosure, there is provided a phototherapy apparatus including a treatment site detection unit, a treatment unit, and a controller. The treatment site detection unit detects a treatment site in a user's body. The treatment unit includes a first moving unit movable in a vertical direction, a body mounted on the first moving unit, a light source unit including multiple light sources disposed on a lower surface of the body and emitting therapeutic light. The controller controls operation of the first moving unit and the light source unit. When the treatment site detection unit detects the treatment site, the controller controls the first moving unit to bring the light source unit into close contact with the treatment site. In addition, when the light source unit closely contacts the treatment site, the controller controls the light source unit such that the light source positioned at the treatment site emits the therapeutic light. When the light source unit closely contacts the treatment site, the body of the treatment unit is deformed by pressure of the treatment site against the multiple light sources. In addition, when the light source unit is separated from the treatment site, the body of the treatment unit is returned to an original shape thereof.

### [Advantageous Effects]

The phototherapy apparatus according to embodiments of the present disclosure can deliver therapeutic light only to a treatment site.

In addition, the phototherapy apparatus according to embodiments of the present disclosure can prevent normal body sites other than a treatment site from being exposed to and damaged by therapeutic light.

### [Description of Drawings]

FIG. 1 to FIG. 6 are exemplary views of a phototherapy apparatus according to a first embodiment of the present disclosure.
FIG. 7 to FIG. 10 are exemplary views of a phototherapy apparatus according to a second embodiment of the present disclosure.
FIG. 11 to FIG. 13 are exemplary views of a phototherapy apparatus according to a third embodiment of the present disclosure.
FIG. 14 is an exemplary view of a phototherapy apparatus according to a fourth embodiment of the present disclosure.
FIG. 15 is an exemplary view of a phototherapy apparatus according to a fifth embodiment of the present disclosure.
FIG. 16 to FIG. 20 are exemplary views of a phototherapy apparatus 600 according to a sixth embodiment of the present disclosure.

### [Best Mode]

Hereinafter, embodiments of the present disclosure will be described with reference to the accompanying drawings. It should be understood that the following embodiments are provided for complete disclosure and thorough understanding of the invention by those skilled in the art. Therefore, the present disclosure is not limited to the following embodiments and may be embodied in different ways. It should be noted that the drawings are not to precise scale and may be exaggerated in width, length, and thickness of components for descriptive convenience and clarity only. The same components will be denoted by the same reference numerals and like components will be denoted by like reference numerals throughout the specification.

A phototherapy apparatus according to the present disclosure includes a treatment site detection unit, a treatment unit, and a controller.

The treatment site detection unit detects a treatment site in a user's body.

The treatment unit includes a first moving unit movable in a vertical direction, a body mounted on the first moving unit, a light source unit including multiple light sources disposed on a lower surface of the body and emitting therapeutic light.

The controller controls operation of the first moving unit and the light source unit.

When the treatment site detection unit detects the treatment site, the controller controls the first moving unit to bring the light source unit into close contact with the treatment site.

In addition, when the light source unit closely contacts the treatment site, the controller controls the light source unit such that the light source positioned at the treatment site emits the therapeutic light.

When the light source unit closely contacts the treatment site, the body of the treatment unit is deformed by pressure of the treatment site against the multiple light sources. In addition, when the light source unit is separated from the treatment site, the body of the treatment unit is returned to an original shape thereof.

In one embodiment, the treatment site detection unit may acquire an image of the user's body through photography of the user's body. In addition, the treatment site detection unit may detect the treatment site from the image and may transmit a treatment site signal containing information about the treatment site to the controller.

In another embodiment, the phototherapy apparatus may further include a second moving unit moving the body of the treatment unit in a horizontal direction.

Here, the treatment site detection unit may detect a location of the treatment site from the image. In addition, the treatment site signal may further contain information about the location of the treatment site.

In addition, the controller may control the second moving unit such that the treatment unit is positioned over the treatment site in response to the treatment site signal.

In a further embodiment, the treatment site detection unit may include a first treatment site detection unit and a second treatment site detection unit.

The first treatment site detection unit may acquire an image of the user's body through photography of the user's body. In addition, the first treatment site detection unit may detect a location of the treatment site from the image.

The second treatment site detection unit may detect the treatment site by receiving light reflected from the user's body. The second treatment site detection unit may be disposed in the light source.

Here, the phototherapy apparatus may further include a second moving unit moving the body of the treatment unit in a horizontal direction.

In addition, the controller may control the second moving unit such that the treatment unit is positioned over the treatment site in response to the treatment site signal.

The light source may include a substrate and a light emitting chip disposed on the substrate.

In yet another embodiment, the light source may further include a body detection unit disposed on the substrate.

An upper surface of the body detection unit may be flush with or higher than an upper surface of the light source.

The controller may stop operation of the first moving unit in response to a body detection signal from the body detection unit.

In addition, the controller may control the light source unit to deliver the therapeutic light to the treatment site upon receiving the body detection signal.

In yet another embodiment, the phototherapy apparatus may further include a temperature sensor detecting a temperature at or around the treatment site. The temperature sensor may transmit a temperature signal to the controller when the detected temperature is higher than or equal to a predetermined value.

The temperature sensor is disposed inside each of the light sources.

The controller may control the light source unit to stop emission of the therapeutic light in response to the temperature signal.

Alternatively, the controller may control the light source unit to stop emission of the therapeutic light from a light source in which a temperature sensor having generated the temperature signal is disposed.

The phototherapy apparatus may further include a housing having a treatment space into which the user's body including the treatment site is inserted.

In addition, the phototherapy apparatus may further include a heat dissipation unit disposed inside the housing and dissipating heat from the treatment space.

In yet another embodiment, the treatment site detection unit may include a wound detection unit detecting a wound site in the user's body and an infection detection unit detecting an infected site corresponding to the treatment site.

The infection detection unit may detect the infected site in the wound site.

The wound detection unit may include a first measurement light source emitting light for detection of the wound site and a first light receiving source receiving light emitted from the wound site by excitation of the light for detection of the wound site.

The infection detection unit may include a second measurement light source emitting light for detection of the infected site and a second light receiving source receiving light emitted from the infected site by excitation of the light for detection of the infected site.

The phototherapy apparatus may further include a display unit displaying the treatment site.

The phototherapy apparatus according to the present disclosure is an apparatus for performing treatment on a treatment site using therapeutic light. The phototherapy apparatus includes a treatment space in which phototherapy is performed. A user's body including a treatment site is inserted into the treatment space.

The phototherapy apparatus according to the present disclosure will be described by way of an example in which the user's body inserted into the treatment space of the phototherapy apparatus is a toe and the treatment site is a toenail. However, the toe and the toenail are intended as an example to aid in understanding of the phototherapy apparatus according to the present disclosure and are not to be construed in any way as limiting the present disclosure.

The phototherapy apparatus according to the present disclosure is applicable to any body site so long as the body site is suitable for phototherapy.

Hereinafter, the phototherapy apparatus according to the present disclosure will be described in detail with reference to the drawings.

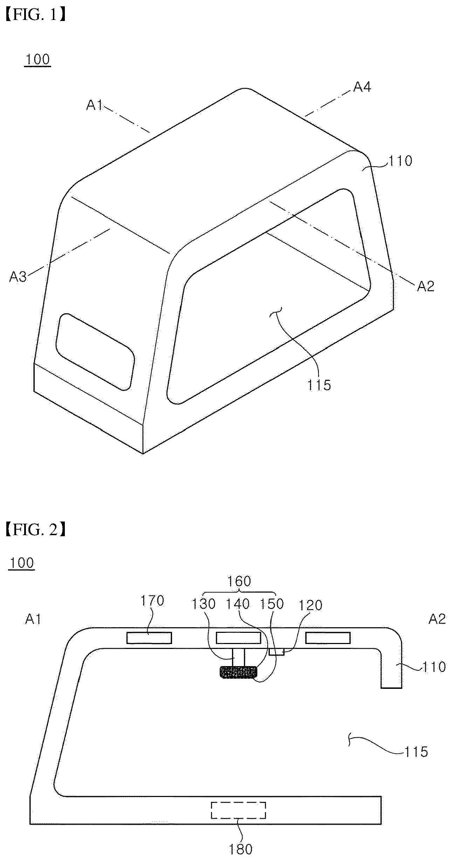

FIG. 1 to FIG. 6 are exemplary views of a phototherapy apparatus according to a first embodiment of the present disclosure.

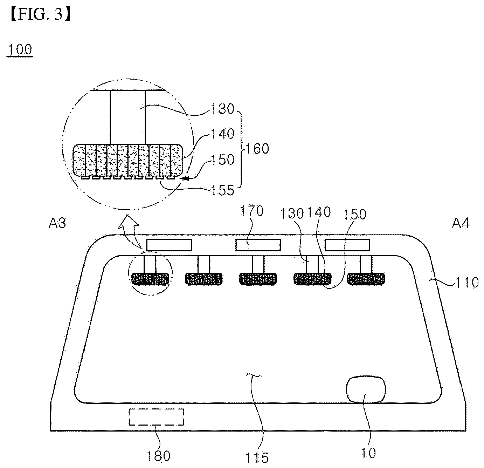

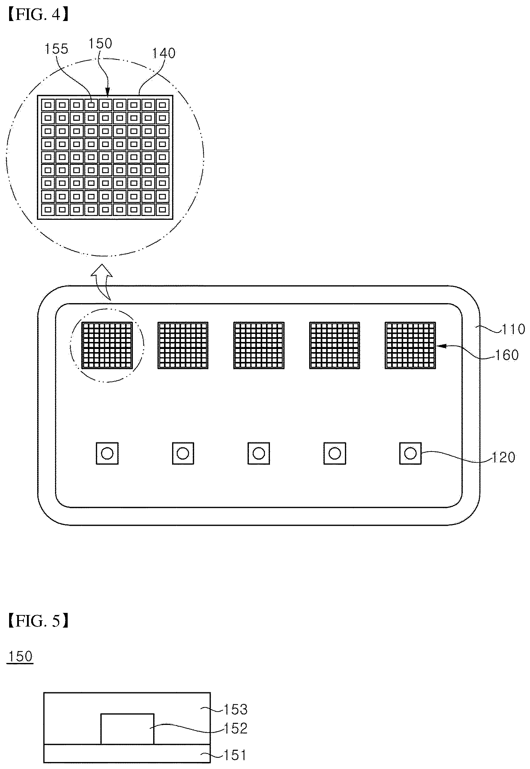

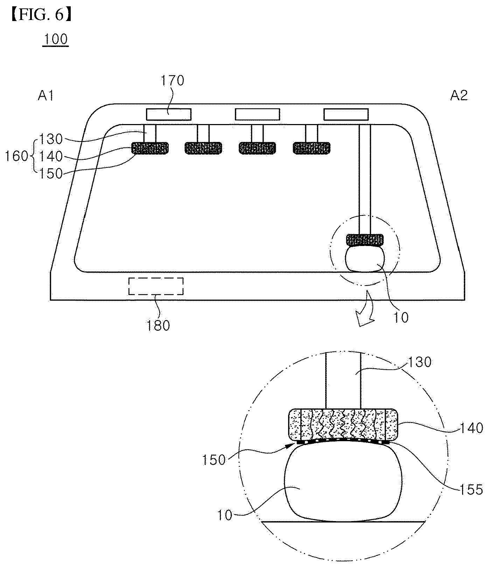

FIG. 1 is a perspective view of the phototherapy apparatus according to the first embodiment. FIG. 2 is a sectional view (A1-A2) of the phototherapy apparatus according to the first embodiment. FIG. 3 is another sectional view (A3-A4) of the phototherapy apparatus according to the first embodiment. FIG. 4 is a view of an inner upper surface of the phototherapy apparatus according to the first embodiment. FIG. 5 is a sectional view of a light source 155 of the phototherapy apparatus according to the first embodiment. FIG. 6 is a view illustrating some operations of the phototherapy apparatus according to the first embodiment.

The phototherapy apparatus 100 according to the first embodiment of the present disclosure includes a housing 110, a treatment site detection unit 120, a treatment unit 160, a controller 180, and a heat dissipation unit 170. The treatment site detection unit 120, the treatment unit 160, the controller 180, and the heat dissipation unit 170 are disposed on the housing 110.

The housing 110 provides a treatment space 115 of the phototherapy apparatus 100. The treatment space 115 is a space in which phototherapy is applied to a treatment site.

The housing 110 has an entrance connecting the treatment space 115 defined in the housing 110 to an outside of the housing 110. A user's body is inserted into the treatment space 115 through the entrance of the housing 110.

The treatment site detection unit 120 detects a treatment site in the user's body. For example, the treatment site detection unit 120 is an imaging device.

According to this embodiment, the treatment site detection unit 120 is disposed on an inner upper surface of the housing 110 facing the treatment space 115.

The treatment site detection unit 120 acquires an image of the user's body placed in the treatment space 115 through photography of the user's body. In addition, the treatment site detection unit 120 detects the treatment site from the acquired image.

The treatment site detection unit 120 may be operated in response to a signal indicating insertion of the user's body into the treatment space 115. The signal may be transmitted from another component detecting insertion of the user's body into the treatment space 115 to the treatment site detection unit 120 directly or through the controller 180. Alternatively, the treatment site detection unit 120 may be operated at the same time as power is supplied to the phototherapy apparatus 100.

When a toe 10 is placed in the treatment space 115, as shown in FIG. 3, the treatment site detection unit 120 photographs the toe 10. The treatment site detection unit 120 may detect a region corresponding to a toenail, which is the treatment site, from an image of the toe 10.

The treatment site detection unit 120 generates a treatment site signal containing information about the detected treatment site. Here, the treatment site-related information may include a region of the image corresponding to the treatment site, a location of the treatment site, and a location of the boundary between the treatment site and other normal sites.

The treatment unit 160 includes a first moving unit 130, a body 140, and a light source unit 150.

The first moving unit 130 is disposed on the inner upper surface of the housing 110 facing the treatment space 115.

The first moving unit 130 is adjustable in length in a vertical direction. That is, one end of the first moving unit 130 is movable up or down by changing the length of the first moving unit 130. For example, the length of the first moving unit 130 may be adjusted by folding or unfolding a portion of the first moving unit 130. Alternatively, the length of the first moving unit 130 may be adjusted by inserting a portion of the first moving unit 130 into another portion of the first moving unit 130 or withdrawing the inserted portion downward. It will be understood that the present disclosure is not limited thereto and the length of the first moving unit 130 may be adjusted in various other ways.

The body 140 is disposed on a lower surface of the first moving unit 130.

The body 140 is connected to the first moving unit 130. For example, the body 140 may be coupled to the lower end of the first moving unit 130. The body 140 is moved up or down by the first moving unit 130. When the first moving unit 130 is extended in length, the body 140 is moved downward. When the extended first moving unit 130 is returned to an original position thereof, the body 140 is moved upward.

The body 140 is formed of an elastically deformable material. The body 140 having elastic deformability is deformed by external force and is returned to an original shape thereof when the force is removed. For example, the body 140 may be formed of an elastic material such as rubber and polyurethane.

Referring to FIG. 3 and FIG. 4, the light source unit 150 is disposed on a lower surface of the body 140. In addition, the light source unit 150 includes multiple light sources 155 emitting therapeutic light. Here, the therapeutic light is light capable of providing removal of an infectious agent from the treatment site or alleviation of lesions. For example, the therapeutic light may be germicidal UV light. Alternatively, the therapeutic light may be visible light having a wavelength of 380 nm to 495 nm, which is near the UV spectrum. Alternatively, the therapeutic light may be infrared light. Alternatively, the therapeutic light may be light including at least one selected from among infrared light, UV light, and visible light.

Referring to FIG. 5, the light source 155 includes a substrate 151, a light emitting chip 152, and a cover 153. The substrate 151 may be any type of substrate 151 that can support the light emitting chip 152. For example, the substrate 151 may be a substrate 151 with a circuit pattern electrically connected to the light emitting chip 152.

The light emitting chip 152 may be a light emitting diode (LED). For example, the light emitting chip 152 may emit at least one selected from infrared, UV, and visible light, as the therapeutic light.

The cover 153 is formed of a material transmitting the light from the light emitting chip 152 therethrough and covers the light emitting chip 152.

The cover 153 has a light incident surface through which the light from the light emitting chip 152 enters the cover and a light exit surface through which the light from the light emitting chip 152 exits the cover.

In one embodiment, the light incident surface of the cover 153 may adjoin the light emitting chip 152, as shown in FIG. 5. That is, the cover 153 may fill a space between the light emitting chip and the light exit surface. For example, the cover 153 may be formed of a silicone resin or an epoxy resin.

In another embodiment, the light incident surface of the cover 153 may be spaced apart from the light emitting chip 152. For example, the cover 153 may be formed of quartz or glass. Here, a space between the cover 153 and the light emitting chip 152 may be empty or may be filled with a light-transmissive resin.

The cover 153 protects the light emitting chip 152 from external foreign substances, such as dust and moisture, and external impact. However, the cover 153 may not be included in the light source 155 and may be omitted as needed.

The light source 155 may further include a wavelength conversion material (not shown) converting a wavelength of the light emitted from the light emitting chip 152.

The wavelength conversion material converts the wavelength of the light emitted from the light emitting chip 152 into a wavelength suitable for a specific purpose.

The body 140 and the light source unit 150 are moved downward by the first moving unit 130. As the body 140 and the light source unit 150 are moved downward, at least some of the multiple light sources 155 contact the user's body. Here, the body 140 is deformed by pressure of the user's body against the multiple light sources. As a result, the multiple light sources 155 closely contact a surface of the user's body. For example, the multiple light sources 155 closely contact a surface of the toe 10, as shown in FIG. 6. In this way, the phototherapy apparatus 100 according to this embodiment can deliver the therapeutic light to the treatment site with the multiple light sources 155 closely contacting the user's body.

The controller 180 controls the overall operation of the phototherapy apparatus 100. The controller 180 controls operation of the first moving unit 130 and the light source unit 150 based on the treatment site-related information detected by the treatment site detection unit 120.

The heat dissipation unit 170 dissipates heat from the treatment space 115 of the housing 110. Accordingly, the heat dissipation unit 170 can prevent heat-induced damage to the user's body inserted into the treatment space 115. In addition, the heat dissipation unit 170 can prevent deterioration in performance of the components disposed on the housing 110 through heat dissipation from the components. For example, the heat dissipation unit 170 may be any known device that can provide heat dissipation, such as a fan and a heatsink.

Next, a phototherapy operation of the phototherapy apparatus 100 according to this embodiment will be described in detail.

The treatment site detection unit 120 acquires an image of a user's body placed in the treatment space 115 through photography of the user's body. In addition, the treatment site detection unit 120 detects a treatment site from the image and transmits a treatment site signal to the controller 180.

In response to the treatment site signal, the controller 180 transmits a vertical movement signal to the first moving unit 130.

In response to the vertical movement signal, the moving unit 130 performs a length extension operation. When the first moving unit 130 performs the length extension operation, the body 140 and the light source unit 150 connected to the first moving unit 130 are moved downward. The first moving unit 130 stops the length extension operation when the length of the first moving unit 130 is extended to the maximum extent or when the length of the first moving unit 130 is no longer extended due to the user's body located under the first moving unit 130.

When the first moving unit 130 stops the length extension operation, at least some of the multiple light sources 155 closely contact a surface of the user's body. Here, the user's body which the light sources 155 closely contact includes a treatment site. For example, the multiple light sources 155 closely contact a surface of a toe 10 including a toenail, which is the treatment site.

The controller 180 controls the light source unit 150 based on the treatment site-related information when the first moving unit 130 stops the length extension operation.

The controller 180 selects light sources 155 positioned at locations corresponding to the treatment site.

For example, the controller 180 may determine whether the location of each light source 155 corresponds to the toenail or the skin of the toe 10 based on the treatment site-related information. Accordingly, the controller 180 may select light sources 155 corresponding in location to the toenail.

Alternatively, the controller 180 may select light sources 155 corresponding in location to the boundary between the toenail and the skin and light sources 155 disposed inside the corresponding light sources 155 based on the treatment site-related information.

The controller 180 controls the phototherapy operation of the light source unit 150 through power supply to the selected light sources 155. Accordingly, among the multiple light sources 155 of the light source unit 150, only the light sources 155 closely contacting the treatment site emit the therapeutic light.

Accordingly, the phototherapy apparatus 100 can deliver the therapeutic light only to the treatment site of the user's body placed in the treatment space 115. For example, the phototherapy apparatus 100 according to this embodiment can deliver the therapeutic light only to the toenail while preventing exposure of the skin of the toe 10 to the therapeutic light.

The controller 180 controls the light source unit 150 to stop the phototherapy operation upon lapse of a predetermined period of time after initiation of phototherapy. Here, phototherapy operation time may be preset and stored in the controller 180 or in another component of the phototherapy apparatus 100. Alternatively, the controller 180 may control the light source unit 150 to stop the phototherapy operation in response to an external input signal.

When the phototherapy operation is stopped, the controller 180 transmits a vertical return signal to the first moving unit 130.

In response to the vertical return signal, the first moving unit 130 is returned to an original position thereof. When the first moving unit 130 is returned to the original position thereof, the body 140 and the light source unit 150 are moved upward and separated from the user's body. As the light sources 155 are separated from the user's body, the force applied to the body 140 is removed and the body 140 is returned to an original shape thereof.

With the body 140 having elastic deformability, the phototherapy apparatus 100 according to this embodiment can bring the light sources 155 into close contact with the treatment site. In addition, with the treatment site detection unit 120, the phototherapy apparatus 100 can select light sources 155 positioned at the treatment site and can allow only the selected light sources 155 to emit the therapeutic light. Accordingly, the phototherapy apparatus 100 can allow only the light sources 155 closely contacting the treatment site to emit the therapeutic light, thereby allowing only the treatment site to be exposed to the therapeutic light. Further, the phototherapy apparatus 100 can prevent other normal sites from being damaged due to exposure to the therapeutic light through restriction of delivery of the therapeutic light only to the treatment site.

Referring to FIG. 3 and FIG. 4, the phototherapy apparatus 100 according to this embodiment includes five treatment site detection units 120 and five treatment units 160 disposed in the treatment space 115 to treat the toenail. However, it will be understood that the structure of the phototherapy apparatus 100 is not limited thereto. For example, the phototherapy apparatus 100 may include one treatment site detection unit 120 adapted to detect multiple treatment sites. In addition, the phototherapy apparatus 100 may include one treatment site detection unit 120 and one treatment unit 160 or may include various other numbers of treatment site detection units 120 and treatment units 160

Next, phototherapy apparatuses according to other embodiments of the present disclosure will be described. Description of the same components as in the above embodiment will be omitted or briefly given. For details of the same components as in the above embodiment, refer to description given for the above embodiment.

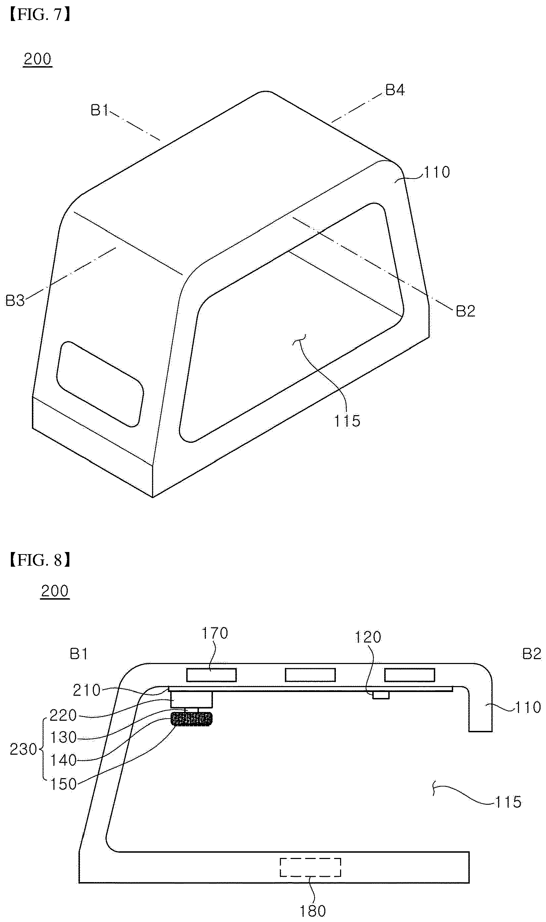

FIG. 7 to FIG. 10 are exemplary views of a phototherapy apparatus according to a second embodiment of the present disclosure.

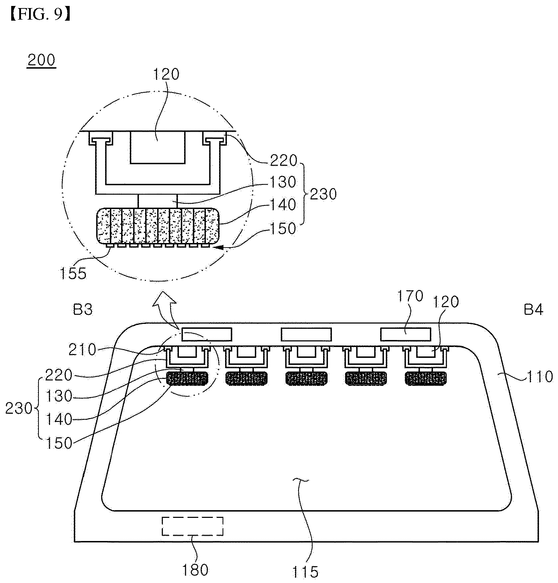

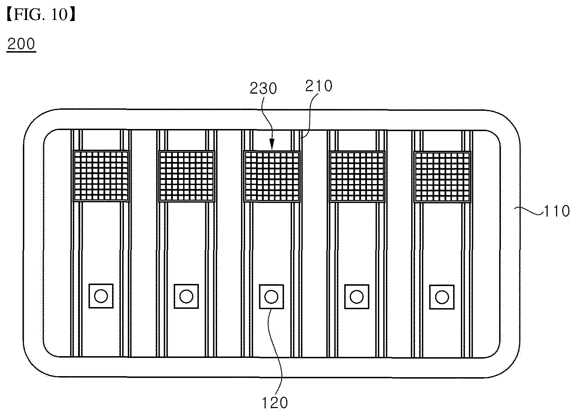

FIG. 7 is a perspective view of the phototherapy apparatus 200 according to the second embodiment. FIG. 8 is a sectional view (B1-B2) of the phototherapy apparatus 200 according to the second embodiment. FIG. 9 is another sectional view (B3-B4) of the phototherapy apparatus 200 according to the second embodiment. FIG. 10 is a view of an inner upper surface of the phototherapy apparatus 200 according to the second embodiment.

The phototherapy apparatus 200 according to the second embodiment includes a housing 110, a treatment site detection unit 120, a second moving unit 210, a treatment unit 230, a controller 180, and a heat dissipation unit 170.

The controller 180 controls a first moving unit 130 and a light source unit 150 of the treatment unit 230 based on treatment site-related information received from the treatment site detection unit 120. In addition, the controller 180 controls the second moving unit 210 based on the treatment site-related information.

The treatment unit 230 includes the first moving unit 130, a body 140, the light source unit 150, and a connection portion 220. The connection unit 220 is connected to the first moving unit 130 and the second moving unit 210.

Referring to FIG. 9, the connection portion 220 is fastened at an upper end to the second moving unit 210. For example, the upper end of the connection portion 220 may be inserted into the second moving unit 210. Alternatively, the connection portion 220 may surround a portion of the second moving unit 210.

In addition, the connection portion 220 is connected at a lower end to the first moving unit 130. The connection portion 220 may be integrally formed with the first moving unit 130. Alternatively, the connection portion 220 may be separately formed from the first moving unit 130 and may be coupled to the first moving unit 130 in various ways.

The treatment unit 230 is coupled to the second moving unit 210 via the connection portion 220 and is horizontally moved by the second moving unit 210.

The second moving unit 210 is disposed on an inner upper surface of the housing 110 facing a treatment space 115.

The second moving unit 210 extends along a line connecting a front surface of the housing 110 to a back surface of the housing 110. The treatment unit 230 is horizontally moved along the second moving unit 210. That is, the second moving unit 210 moves the treatment unit 230 toward the front surface of the housing 110, which is formed with an entrance, or moves the treatment unit 230 toward the back surface of the housing 110, which is opposite the front surface.

The controller 180 generates a horizontal movement signal based on the treatment site-related information received from the treatment site detection unit 120. The horizontal movement signal contains information about a location of a treatment site. For example, the information about the location of the treatment site may include information about a location of the center of the treatment site. Alternatively, the information about the location of the treatment site may include information about a location of the boundary between the treatment site and other normal sites. As such, the information about the location of the treatment site may include any type of information that allows identification of the location of the treatment site.

The controller 180 transmits the horizontal movement signal containing the information about the location of the treatment site to the second moving unit 210.

In response to the horizontal movement signal, the second moving unit 210 moves the treatment unit 230 to a position over the treatment site. Here, the second moving unit 210 may move the treatment unit 230 such that a lower surface of the treatment unit 230 is positioned over the entire treatment site.

When the phototherapy apparatus 200 according to this embodiment includes multiple treatment units 230, the phototherapy apparatus 200 may include the same number of second moving units 210 as the number of treatment units 230 such that each of the treatment units 230 can be moved to a different position. Accordingly, the multiple second moving units 210 may be connected to respective treatment units 230 to individually move each of the multiple treatment units 230.

When the second moving unit 210 is positioned over the treatment site, the controller 180 generates a vertical movement signal and transmits the vertical movement signal to the first moving unit 130.

In response to the vertical movement signal, the first moving unit 130 is operated to be extended in length. As the first moving unit 130 is extended in length, the body 140 and the light source unit 150 are moved downward.

When the light source unit 150 closely contacts the treatment site, the controller 180 controls the light source unit 150 such that a light source 155 closely contacting the treatment site emits the therapeutic light.

After completion of phototherapy, the controller 180 generates a vertical return signal and transmits the vertical return signal to the first moving unit 130.

When the first moving unit 130 is returned to an original position thereof, the controller 180 generates a horizontal return signal and transmits the horizontal return signal to the second moving unit 210.

In response to the horizontal return signal, the second moving unit 210 is returned to an original position thereof.

Alternatively, the controller 180 may simultaneously transmit the vertical return signal and the horizontal return signal to the first moving unit 130 and the second moving unit 210, respectively.

The phototherapy apparatus 200 according to this embodiment detects the location of the treatment site using the treatment site detection unit 120 and moves the treatment unit 230 to a position over the detected treatment site using the second moving unit 210. Accordingly, the phototherapy apparatus 200 allows the treatment unit 230 to cover the entire treatment site in a more accurate manner than when a user personally positions the treatment site under the treatment unit 230. Thus, the phototherapy apparatus 200 allows phototherapy to be applied to the entire treatment site at the same time.

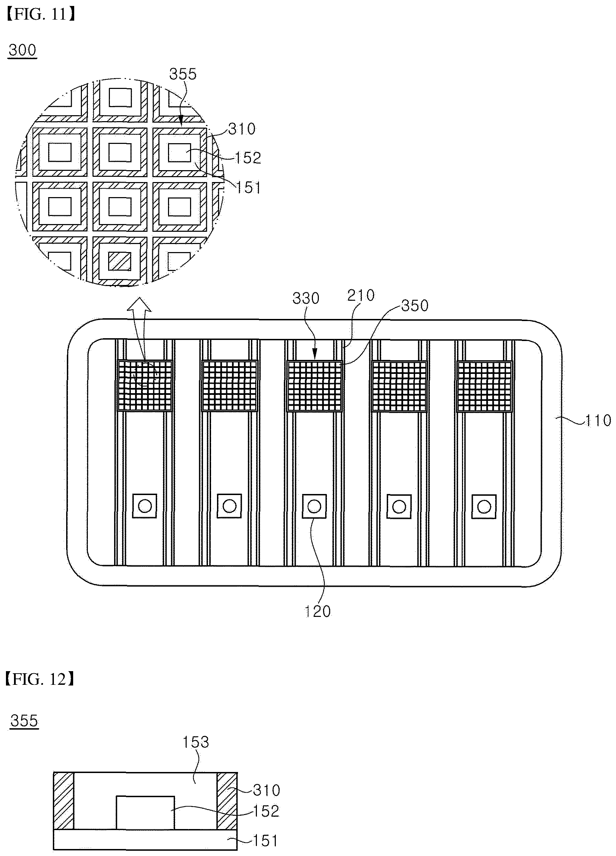

FIG. 11 to FIG. 13 are exemplary views of a phototherapy apparatus according to a third embodiment of the present disclosure.

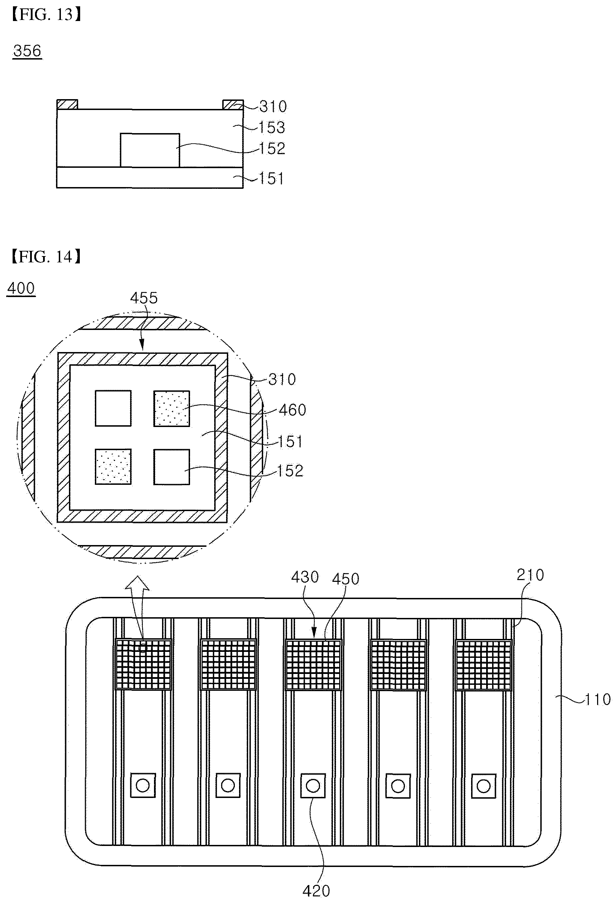

FIG. 11 is a view of an inner upper surface of the phototherapy apparatus 300 according to the third embodiment. FIG. 12 is a sectional view of one exemplary light source 355 of the phototherapy apparatus 300 according to the third embodiment. FIG. 13 is a sectional view of another exemplary light source 356 of the phototherapy apparatus 300 according to the third embodiment.

The phototherapy apparatus 300 according to the third embodiment includes the housing 110, the treatment site detection unit 120, the second moving unit 210, a treatment unit 330, the controller 180, and the heat dissipation unit 170.

The treatment unit 330 includes a first moving unit 130, a body 140, and a light source unit 350 including multiple light sources 355.

In this embodiment, each of the multiple light sources 355 includes a substrate 151, at least one light emitting chip 152, a cover 153, and a body detection unit 310. The body detection unit 310 detects contact with the user's body.

Referring to FIG. 11, the body detection unit 310 is disposed around an edge of the light source 355. In addition, the body detection unit 310 is provided to each light source 355.

Referring to FIG. 12, the one exemplary light source 355 has a structure in which the body detection unit 310 is disposed on an upper surface of the substrate 151 and covers a side surface of the cover 153. Although the light source 355 has been described to include the cover 153 in this embodiment, it will be understood that the present disclosure is not limited thereto and the cover 153 may be omitted.

A light source constituting the light source unit 350 of the phototherapy apparatus 300 according to this embodiment is not limited to the light source 355 of FIG. 12. A light source constituting the light source unit 350 may be the other exemplary light source 356 of FIG. 13. The light source 356 of FIG. 13 has a structure in which the body detection unit 310 is disposed on an upper surface of the cover 153.

As such, an upper surface of the body detection unit 310 may be flush with or higher than an upper surface of the light emitting chip 152. In addition, the upper surface of the body detection unit 310 may be flush with or higher than an upper surface of the upper surface of the cover 153. Accordingly, when the treatment unit 330 is moved downward to perform phototherapy, the body detection unit 310 contacts the user's body placed in the treatment space 115.

In response to a treatment site signal from the treatment site detection unit 120, the controller 180 controls at least one of the first moving unit 130 and the second moving unit 210 such that the treatment unit 330 closely contacts the treatment site.

Upon contact with the user's body, the body detection unit 310 of each light source 355 generates a body detection signal and transmits the body detection signal to the controller 180.

In response to the body detection signals from the light sources 355, the controller 180 stops the length extension operation of the first moving unit 130 to stop downward movement of the treatment unit 330.

For example, when the controller 180 receives the body detection signal greater than or equal to a predetermined value, the controller 180 may determine that the entire treatment site closely contacts the light sources 355. Here, the predetermined value may be the number of body detection units 310 generating the body detection signal received by the controller 180.

The controller 180 may determine the degree of close contact between the user's body and the light source unit 350 through identification of the number of light sources 355 having generated the body detection signal. Here, the light source 355 having generated the body detection signal is a light source 355 including a body detection unit 310 having generated the body detection signal. Further, the controller 180 may determine whether the entire treatment site closely contacts the light sources 355 based on the degree of close contact between the user's body and the light source unit 350.

For example, when the controller 180 receives the body detection signal from 95% or more of all the light sources 355, the controller 180 may stop the length extension operation of the first moving unit 130.

Alternatively, the controller 180 may determine the degree of close contact between the treatment site and the light source unit 350 through comparison of the treatment site-related information with the received body detection signal. When the controller 180 receives the body detection signal from all the body detection units 310 of light sources 355 positioned in a region corresponding to the treatment site, the controller 180 may determine that the entire treatment site closely contacts the light sources 355. When the controller 180 receives the body detection signal from all the light sources 355 closely contacting the treatment site, the controller 180 may stop the length extension operation of the first moving unit 130.

Thereafter, the controller 180 controls the light source unit 350 to perform phototherapy.

The phototherapy apparatus 300 according to this embodiment delivers the therapeutic light to the treatment site after the controller 180 receives both the treatment site signal and the body detection signal. Accordingly, the phototherapy apparatus 300 according to this embodiment can prevent the light source unit 350 from emitting the therapeutic light before the treatment unit 330 closely contacts the treatment site. Thus, the phototherapy apparatus 300 according to this embodiment can prevent the therapeutic light from being delivered to a body site other than the treatment site by allowing the therapeutic light to be emitted after the treatment unit 330 closely contacts the entire treatment site.

In addition, the phototherapy apparatus 300 according to this embodiment can detect a point in time when the treatment unit 330 closely contacts the entire treatment site. Accordingly, the phototherapy apparatus 300 according to this embodiment can adjust the range of downward movement of the treatment unit 330 depending on the height of the treatment site inserted into the treatment space 115. Thus, the phototherapy apparatus 300 according to this embodiment can prevent user discomfort due to intense pressure of the treatment unit 330 against the user's body including the treatment site.

FIG. 14 is an exemplary view of a phototherapy apparatus according to a fourth embodiment of the present disclosure.

FIG. 14 is a view of an inner upper surface of the phototherapy apparatus 400 according to the fourth embodiment.

The phototherapy apparatus 400 according to the fourth embodiment includes a housing 110, a treatment site detection unit, a second moving unit 210, a treatment unit 430, a controller 180, and a heat dissipation unit 170.

According to this embodiment, the treatment site detection unit includes a first treatment site detection unit 420 detecting a location of a treatment site and a second treatment site detection unit 460 detecting the treatment site.

For example, the first treatment site detection unit 420 may include a photographing device and the second treatment site detection unit 460 may include an optical sensor. In addition, the second treatment site detection unit 460 may be disposed inside each light source 455.

The first treatment site detection unit 420 photographs a user's body inserted into the treatment space 115. In addition, the first treatment site detection unit 420 detects the location of the treatment site based on an acquired image of the user's body. The first treatment site detection unit 420 transmits a treatment site location signal containing information about the location of the treatment site to the controller 180.

In response to the treatment site location signal, the controller 180 controls the first moving unit 130 and the second moving unit 210 such that the treatment unit 430 closely contacts the user's body including the treatment site. Here, the controller 180 may stop the length extension operation of the first moving unit 130 upon receiving a body detection signal from a body detection unit 310 of the treatment unit 430.

When the operation of the first moving unit 130 is stopped, the second treatment site detection unit 460 starts a treatment site detection operation. For example, upon receiving a treatment site detection signal from the controller 180, the second treatment site detection unit 460 may start the treatment site detection operation.

Alternatively, the second treatment site detection unit 460 may start the treatment site detection operation when the body detection signal is generated from a light source 455 in which the second treatment site detection unit 460 is disposed.

The second treatment site detection unit 460 receives light reflected from the user's body. For example, the second treatment site detection unit 460 delivers detection light to the user's body and receives the detection light reflected from the user's body. The second treatment site detection unit 460 may determine whether a body site closely contacting the light source 455 is the treatment site or a normal site based on calculation of a reflectance with respect to the detection light. Here, the detection light is light used to determine whether the body site is the treatment site or the normal site. For example, the detection light may be visible light having a specific wavelength.

Different sites in the body absorb different amounts of light. That is, different sites in the body can have different reflectance values. In addition, there can be a difference in reflectance between a normal body site and a body site in which an infectious agent is present. For example, the skin of the toe has a different reflectance than the toenail. Accordingly, the second treatment site detection unit 460 calculates a reflectance with respect to the detection light and compares the calculated reflectance with a predetermined value. Here, the predetermined value is a reflectance measured at the treatment site. When the calculated reflectance corresponds to the predetermined value, the second treatment site detection unit 460 generates a treatment site detection signal and transmits the treatment site detection signal to the controller 180.

Upon receiving the treatment site detection signal, the controller 180 determines that the light source 455 including the second treatment site detection unit 460 having generated the treatment site detection signal is positioned at the treatment site.

Each treatment site detection unit may include multiple second treatment site detection units 460. For example, two second treatment site detection units 460 may be disposed inside each light source 455, as shown in FIG. 14.

Each of the multiple second treatment site detection units 460 disposed inside each light source 455 may perform a treatment site detection operation. The multiple second treatment site detection units 460 may produce different detection results depending on which body site the second treatment site detection unit faces. For example, when all the light sources 455 are positioned at the treatment site, all the multiple second treatment site detection units 460 transmit the treatment site detection signal to the controller 180. When the light source 455 is positioned at the boundary between the treatment site and a normal site, only one second treatment site detection unit 460 may transmit the treatment site detection signal to the controller 180.

Provided that the controller 180 receives the treatment site detection signal from all the second treatment site detection units 460 disposed in one light source 455, the controller 180 determines that the light source 455 is positioned at the treatment site.

The controller 180 may control the light source unit 450 such that the light source 455 determined to be positioned at the treatment site emits the therapeutic light.

With the second treatment site detection unit 460 disposed inside each light source 455, the phototherapy apparatus 400 according to this embodiment determines whether a body site closely contacting a corresponding light source 455 is the treatment site. Accordingly, the phototherapy apparatus 400 can more precisely select light sources 455 positioned at the treatment site.

In addition, with the multiple second treatment site detection units 460 disposed inside each light source 455, the phototherapy apparatus 400 according to this embodiment can select light sources 455 closely contacting only the treatment site.

Thus, the phototherapy apparatus 400 according to this embodiment can restrict delivery of the therapeutic light to the exact treatment site through precise selection of light sources 455 closely contacting the treatment site.

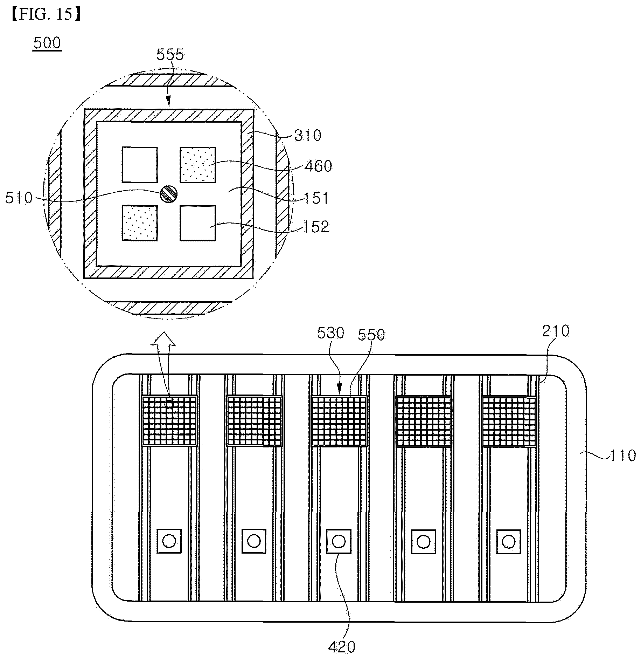

FIG. 15 is an exemplary view of a phototherapy apparatus according to a fifth embodiment of the present disclosure.

Referring to FIG. 15, the phototherapy apparatus 500 according to the fifth embodiment includes a housing 110, a treatment site detection unit, a second moving unit 210, a treatment unit 530, a controller 180, and a heat dissipation unit 170. The treatment site detection unit may include a first treatment site detection unit 420 and a second treatment site detection unit 460.

The treatment unit 530 includes a first moving unit 130, a body 140, and a light source unit 550 including multiple light sources 555.

In this embodiment, each of the multiple light sources 555 includes a substrate 151, at least one light emitting chip 152, a cover 153, a body detection unit 310, and a temperature sensor 510. The temperature sensor 510 may be provided to each light source 555.

When the light source 555 delivers the therapeutic light to a treatment site, the temperature at the treatment site may be increased depending on the wavelength, intensity, and exposure time of the therapeutic light.

The temperature sensor 510 detects the temperature around the light source 555 and a user's body. Upon detecting a temperature higher than or equal to a predetermined value, the temperature sensor 510 transmits a temperature signal to the controller 180.

In response to the temperature signal, the controller 180 may control the light source unit 550 such that all the light sources 555 stop emitting the therapeutic light.

Alternatively, the controller 180 may control the light source unit 550 such that a corresponding light source 555 stops emitting the therapeutic light in response to the temperature signal. Here, the corresponding light source 555 is a light source 555 including a temperature sensor 510 having generated the temperature signal.

Accordingly, the phototherapy apparatus 500 according to this embodiment can prevent damage to the user's body due to the therapeutic light delivered to the treatment site. For example, the phototherapy apparatus 500 can prevent a user from being burned by the therapeutic light or experiencing discomfort due to high temperature.

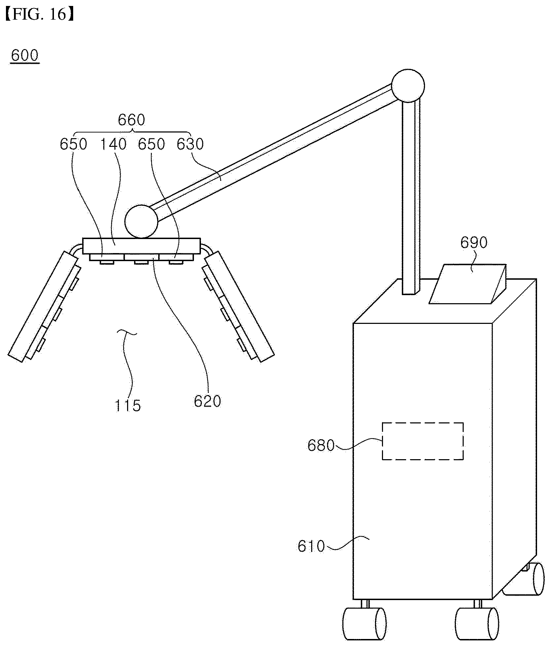

FIG. 16 to FIG. 20 are exemplary views of a phototherapy apparatus 600 according to a sixth embodiment of the present disclosure.

Referring to FIG. 16 to FIG. 20, the phototherapy apparatus 600 according to the sixth embodiment includes a treatment site detection unit 620, a treatment unit 660, a controller 680, and a display unit 690.

The treatment site detection unit 620 includes a wound detection unit 621 and an infection detection unit 625.

The wound detection unit 621 detects a wound on the skin and a location of a wound site 11. The wound detection unit 621 includes a first measurement light source 622 and a first light receiving source 623. The first measurement light source 622 may include a substrate and a light emitting chip emitting light for wound detection.

The wound detection unit 621 emits light having a specific wavelength and receives light emitted from a wound by excitation of the light.

An open wound exposes dermal tissue of the skin to an outside environment. When the dermal tissue of the skin is exposed to the outside environment, fibrous proteins present in the dermis are also exposed to the outside environment. Here, examples of the fibrous proteins include collagen, elastin, and the like.

For example, collagen absorbs light having a wavelength of 330 nm to 340 nm and emits light having a wavelength of 400 nm to 410 nm by excitation of the absorbed light. In addition, elastin absorbs light having a wavelength of 350 nm to 420 nm and emits light having a wavelength range of 420 nm to 510 nm by excitation of the absorbed light.

Accordingly, the first measurement light source 622 may emit light having a wavelength of 330 nm to 340 nm and the first light receiving source 623 may receive light having a wavelength of 400 nm to 410 nm. Alternatively, the first measurement light source 622 may emit light having a wavelength of 350 nm to 420 nm and the first light receiving source 623 may receive light having a wavelength range of 410 nm to 510 nm. Alternatively, the first measurement light source 622 may emit light having a wavelength range of 330 nm to 420 nm and the first light receiving source 623 may receive light having a wavelength range of 400 nm to 510 nm.

In this way, the wound detection unit 621 detects the location and extent of the wound site 11 through detection of the exposed fibrous proteins.

The infection detection unit 625 detects an infected site 12 in the wound site 11. That is, the infection detection unit 625 detects an infectious agent present in the wound site 11. The infection detection unit 625 includes a second measurement light source 626 and a second light receiving source 627. The second measurement light source 626 may include a substrate and a light emitting chip emitting light for detection of the infected site 12.

The infection detection unit 625 emits light having a specific wavelength and receives light emitted from the infectious agent by excitation of the incident light.

Porphyrin is a necessary element for organic respiration of infectious agents such as bacteria. Porphyrin absorbs light having a specific wavelength and emits light by excitation of the absorbed light.

For example, porphyrin absorbs light having a wavelength of 405 nm and emits light having a wavelength of 635 nm by excitation of the absorbed light. Alternatively, porphyrin absorbs light having a wavelength of 635 nm and emits light having a wavelength of 705 nm by excitation of the absorbed light.

Accordingly, the second measurement light source 626 may emit light having a wavelength of 405 nm and the second light receiving source 627 may receive light having a wavelength of 635 nm. Alternatively, the second measurement light source 626 may emit light having a wavelength of 635 nm and the second light receiving source 627 may receive light having a wavelength of 705 nm. Alternatively, the second measurement light source 626 may emit light having wavelengths of 405 nm and 635 nm and the second light receiving source 627 may receive light having wavelengths of 635 nm and 705 nm.

Porphyrin produces active oxygen through absorption of light. When the concentration of active oxygen is low, cells of an infectious agent proliferate. When the concentration of active oxygen is high, an infectious agent stops cell division and cells of the infectious agent die due to activation of a substance involved in cell death.

Accordingly, the infection detection unit 625 may serve not only to detect the infection site 12 but also to kill cells of an infectious agent.

In order to accurately detect the wound site 11 and the infected site 12 in the wound site 11, it is desirable that light used in the wound detection unit 621 have a different wavelength from light used in the infection detection unit 625. Accordingly, in this embodiment, the first measurement light source 622 of the wound detection unit 621 emits light having a wavelength of 330 nm to 340 nm and the first light receiving source 623 of the wound detection unit 621 emits light having a wavelength of 400 nm to 410 nm, whereas the second measurement light source 626 of the infection detection unit 625 emits light having a wavelength of 635 nm and the second light receiving source 627 of the infection detection unit 625 receives light having a wavelength of 705 nm.

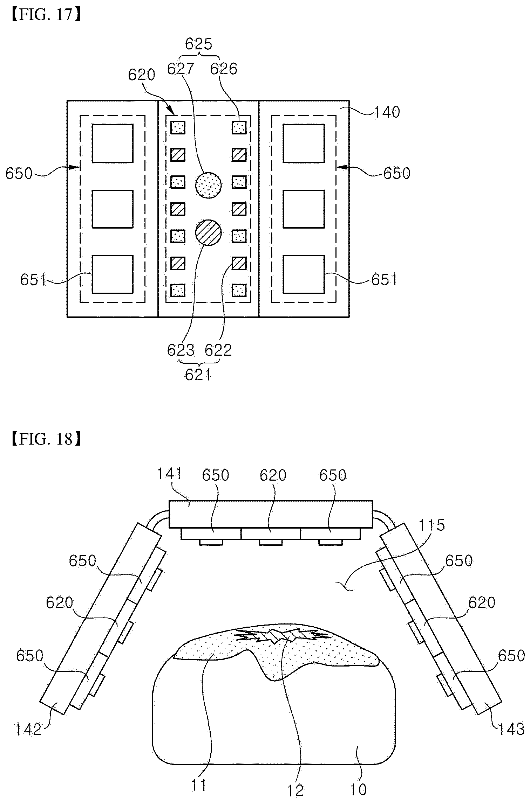

Although the treatment site detection unit 620 is shown as including one first light receiving source 623 and one second light receiving source 627 in FIG. 17, it will be understood that the present disclosure is not limited thereto and the treatment site detection unit 620 may include multiple first light receiving sources 623 and multiple second light receiving sources 627.

The treatment unit 660 includes a moving unit 630, a body 140, and a light source unit 650.

The light source unit 650 is disposed on a lower surface of the body 140. The light source unit 650 includes multiple light sources 651. Each of the light source 651 may include a light emitting chip emitting therapeutic light capable of killing an infectious agent present at a wound. For example, the light source 651 may emit at least one selected from among light having a wavelength of 200 nm to 280 nm, which is germicidal UVC light, and light having a wavelength of 280 nm to 320 nm, which is UVB light.

Alternatively, the light source 651 may emit therapeutic light capable of causing death of the infectious agent through increase in concentration of active oxygen in the infectious agent. For example, the light source 651 may emit at least one selected from among light having a wavelength of 405 nm and light having a wavelength of 635 nm, which are absorbable by porphyrin.

Alternatively, the light source 651 may emit both light capable of killing the infectious agent and light capable of increasing the concentration of active oxygen in the infectious agent.

Although the light source unit 650 is shown as including multiple light sources in FIG. 17, it will be understood that the present disclosure is not limited thereto. The light source unit 650 may have a structure in which multiple light emitting chips are mounted on a single substrate.

The moving unit 630 is connected to an upper surface of the body 140. In addition, one end of the moving unit 630 is moved up or down in response to a signal from the controller 680. Here, the one end of the moving unit 630 is a portion at which the moving unit 630 contacts the body 140. Accordingly, the body 140 and the light source unit 650 may be moved up or down by movement of the moving unit 630.

In addition, the one end of the moving unit 630 connected to the body 140 may be moved right or left in response to a signal from the controller 680. As the one end of the moving unit 630 is moved to right or left, the body 140 and the light source unit 650 are also moved right or left. For example, the moving unit 630 may be an arm as shown in FIG. 16.

According to this embodiment, the treatment site detection unit 620 and the light source unit 650 are disposed on the lower surface of the body 140 of the treatment unit 660. In addition, the wound detection unit 621, the infection detection unit 625, and the light source unit 650 may be configured separately from one another or may share a single substrate.

In addition, the phototherapy apparatus 600 according to this embodiment includes multiple treatment units 660 and multiple treatment site detection units 620. For convenience of description, respective main bodies 140 of the multiple treatment units 660 shown in FIG. 18 are referred to as first to third main bodies 141 to 143. Referring to FIG. 18, the phototherapy apparatus 600 includes first to third main bodies 141 to 143 connected to one another. The light source unit 650 and the treatment site detection unit 620 are disposed on each of the first to third main bodies 141 to 143.

The first body 141 is connected to a lower end of the moving unit 630 with the treatment site detection unit 620 and the light source unit 650 facing downward, and the second body 142 and the third body 143 are disposed at opposite sides of the first body 141. Here, the second body 142 and the third body 143 are disposed such that the respective treatment site detection units 620 and light source units 650 thereof face downwardly of the first body 141. In addition, the second body 142 and the third body 143 may be disposed at varying angles with respect to the first body 141. Accordingly, a treatment space 115 is defined inside the body 140 by the first to third bodies 141 to 143. Accordingly, the phototherapy apparatus 600 can ensure more accurate detection and treatment of the treatment site by varying the location of the treatment site detection unit 620 and the light source unit 650 facing the treatment space 115.

Although the phototherapy apparatus 600 has been described as including three bodies 140, three light source units 650, and three treatment site detection units 620, it will be understood that the present disclosure is not limited thereto. The numbers of bodies 140, light source units 650, and treatment site detection units 620 of the phototherapy apparatus 600 may be varied according to the choice of those skilled in the art.

The controller 680 controls the overall operation of the phototherapy apparatus 600 according to this embodiment.

The controller 680 controls operation of the moving unit 630 and the light source unit 650 based on information about the wound site 11 and the infected site 12 detected by the treatment site detection unit 620. In addition, the controller 680 may control operation of the treatment site detection unit 620.

The display unit 690 displays treatment site-related information. For example, the display unit 690 may display the location and extent of a wound, the shape of the wound site 11, and the like. In addition, the display unit 690 may display the location of the infected site 12, the extent of infection, and the like. The display unit 690 may display the treatment site-related information in the form of an image.

In addition, the display unit 690 may be disposed anywhere so long as the display unit 690 can display the treatment site-related information in the form of an image. For example, the display unit 690 may be disposed on a main body 610 provided with the controller 680, the moving unit 630, and the like. Alternatively, the display unit 690 may be configured as a separate device.

Next, the phototherapy operation of the phototherapy apparatus 600 according to this embodiment will be described.

Referring to FIG. 18, a treatment site is inserted into the treatment space 115. Here, in response to an external signal, the controller 680 may control the wound detection unit 621 to detect the wound site 11 on a toe 10, which is a body site inserted into the treatment space 115. For example, the external signal may be a signal input through the main body 610 of the phototherapy apparatus 600 to instruct start of the phototherapy operation. Alternatively, the external signal may be a signal from a sensor detecting insertion of the body site into the treatment space 115.

When the wound detection unit 621 detects no wound site 11, the controller 680 may stop all operations of the phototherapy apparatus 600.

When the wound detection unit detects a wound site 11, but the wound detection unit fails to detect the entirety of the wound site 11, the controller 680 controls the moving unit 630. For example, when the wound detection unit 621 detects a wound site 11 completely surrounded by normal sites, the controller 680 does not move the moving unit 630. On the contrary, when the wound detection unit 621 detects a wound site partially surrounded by normal sites, the controller 680 may control the moving unit 630 such that the wound detection unit 621 is spaced farther apart from the wound site 11. That is, the controller 680 may control the moving unit 630 to be moved upward such that the wound detection unit 621 can detect the entirety of the wound area 11.

Alternatively, the controller 680 may change the angle of the body 140 located at both sides of the body site such that the wound detection unit 621 is spaced farther apart from the wound site 11.

When the entirety of the wound site 11 is detected, the controller 680 may control the infection detection unit 625 to detect the infected site 12 in the wound site 11.

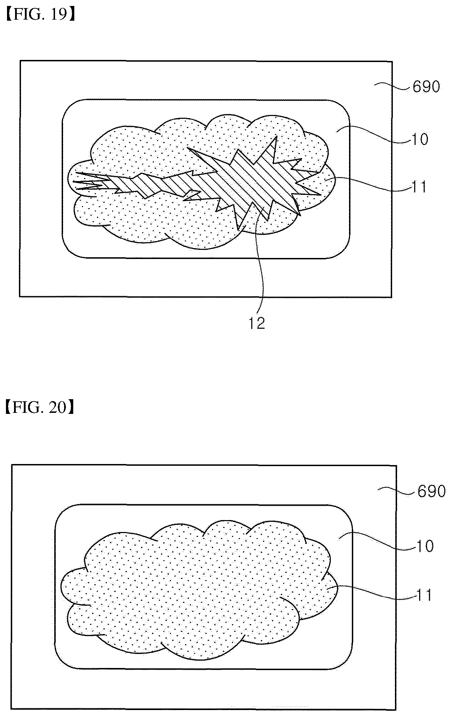

Here, the phototherapy apparatus 600 may display information such as the location, extent, and shape of the wound site 11 and the infected site 12 on the display unit 690. The display unit 690 may display the information about the wound site 11 and the infected site 12 in the form of an image, as shown in FIG. 19. A user may determine the degree of the wound and the degree of infection in the wound site 11 based on the image displayed by the display unit 690.

The controller 680 may control the light source unit 650 to emit the therapeutic light based on infection-related information detected by the infection detection unit 625. The controller 680 may control the light source unit 650 such that a light source corresponding in location to the infected site 12 emits the therapeutic light. Accordingly, the phototherapy apparatus 600 can deliver the therapeutic light only to the infected site 12 detected by the infection detection unit 625.

Here, the controller 680 may control the moving unit 630 such that the light source unit 650 closely contacts the treatment site. In this way, it is possible to reduce the range of illumination with the therapeutic light from each light source, thereby ensuring exact delivery of the therapeutic light to the infected site 12.

When an infectious agent is removed from the infected site 12 by phototherapy, the display unit 690 displays an image of the wound site 11 from which the infectious agent has disappeared, as shown in FIG. 20.

In this way, the phototherapy apparatus 600 can identify the infected site 12 in the wound site 11 and can deliver the therapeutic light primarily to the infected site 12. In addition, the phototherapy apparatus 600 can ensure real time monitoring of the infectious agent removal process through the display unit 690. Accordingly, the phototherapy apparatus 600 according to this embodiment can prevent incomplete treatment of infections due to insufficient phototherapy application time or unnecessary exposure of a user's body to the therapeutic light due to excessive phototherapy application time.

The phototherapy apparatus 600 according to this embodiment may further include other components described above related to the phototherapy apparatuses according to the above embodiments.

Although some embodiments have been described herein, it should be understood that these embodiments are provided for illustration only and are not to be construed in any way as limiting the present disclosure, and that the scope of the present invention is defined by the appended claims .

## Claims

1. A phototherapy apparatus comprising:
a treatment site detection unit for detecting a treatment site in a user's body;
a treatment unit comprising a first moving unit movable in a vertical direction, a body mounted on the first moving unit, a light source unit comprising multiple light sources disposed on a lower surface of the body and emitting therapeutic light; and
a controller for controlling operation of the first moving unit and the light source unit, the controller being configured to control:
upon detection of the treatment site with the treatment site detection unit, the first moving unit to bring the light source unit into contact with the treatment site; and,
upon determination of contact between the light source unit and the treatment site, the light source unit positioned at the treatment site to emit the therapeutic light; and,
wherein the body of the treatment unit is deformable by pressure of the treatment site against the multiple light sources and, upon release of the pressure, the body of the treatment unit is configured to return to an original shape thereof.

2. The phototherapy apparatus according to claim 1, wherein:
the treatment site detection unit is configured to acquire an image of a user's body by way of photography of a user's body; and
the treatment site detection unit is configured to detect the treatment site from the image and to transmit a treatment site signal containing information about the treatment site to the controller.

3. The phototherapy apparatus according to claim 2, further comprising:
a second moving unit for moving the body of the treatment unit in a horizontal direction.

4. The phototherapy apparatus according to claim 3, wherein the treatment site detection unit is configured to detect a location of the treatment site from the image and the treatment site signal further contains information about the location of the treatment site.

5. The phototherapy apparatus according to claim 4, wherein the controller is configured to control the second moving unit such that the treatment unit is positioned over the treatment site in response to the treatment site signal.

6. The phototherapy apparatus according to claim 1, wherein the treatment site detection unit further comprises:
a first treatment site detection unit for acquiring an image of a user's body through photography of a user's body and detecting a location of the treatment site from the image; and
a second treatment site detection unit for detecting the treatment site by receiving light reflected from a user's body.

7. The phototherapy apparatus according to claim 6, further comprising:
a second moving unit for moving the body of the treatment unit in a horizontal direction.

8. The phototherapy apparatus according to claim 7, wherein the controller is configured to control the second moving unit such that the treatment unit is positioned over the treatment site in response to the treatment site signal.

9. The phototherapy apparatus according to claim 6, wherein the second treatment site detection unit is disposed in the light source unit.

10. The phototherapy apparatus according to claim 1, wherein a light source comprises a substrate and a light emitting chip disposed on the substrate.

11. The phototherapy apparatus according to claim 10, wherein the light source further comprises a body detection unit disposed on the substrate.

12. The phototherapy apparatus according to claim 11, wherein an upper surface of the body detection unit is flush with or higher than an upper surface of the light source.

13. The phototherapy apparatus according to claim 11, wherein the controller is configured to stop operation of the first moving unit in response to a body detection signal from the body detection unit.

14. The phototherapy apparatus according to claim 13, wherein the controller is configured to control the light source unit to deliver the therapeutic light to the treatment site upon receiving the body detection signal.

15. The phototherapy apparatus according to claim 1, further comprising:
a temperature sensor for detecting a temperature at or around the treatment site and for transmitting a temperature signal to the controller when the detected temperature is higher than or equal to a predetermined value.

## Patentansprüche

1. Lichttherapievorrichtung mit:
eine Behandlungsstellen-Erfassungseinheit zum Erfassen einer Behandlungsstelle im Körper eines Benutzers;
eine Behandlungseinheit mit einer ersten beweglichen Einheit, die in vertikaler Richtung beweglich ist, einem Körper, der auf der ersten beweglichen Einheit montiert ist, einer Lichtquelleneinheit, die mehrere Lichtquellen umfasst, die auf einer unteren Oberfläche des Körpers angeordnet sind und therapeutisches Licht emittieren; und
eine Steuereinheit zum Steuern des Betriebs der ersten beweglichen Einheit und der Lichtquelleneinheit, wobei die Steuereinheit so konfiguriert ist, dass sie steuert:
bei Erfassung der Behandlungsstelle mit der Behandlungsstellen-Erfassungseinheit die erste bewegliche Einheit, um die Lichtquelleneinheit in Kontakt mit der Behandlungsstelle zu bringen; und,
bei Feststellung des Kontakts zwischen der Lichtquelleneinheit und der Behandlungsstelle die Lichtquelleneinheit an der Behandlungsstelle positioniert, um das therapeutische Licht zu emittieren; und,
wobei der Körper der Behandlungseinheit durch den Druck der Behandlungsstelle gegen die mehreren Lichtquellen verformbar ist und der Körper der Behandlungseinheit so konfiguriert ist, dass er bei Aufhebung des Drucks wieder seine ursprüngliche Form annimmt.

2. Lichttherapievorrichtung nach Anspruch 1, wobei:
die Behandlungsstellen-Erfassungseinheit so konfiguriert ist, dass sie ein Bild des Körpers eines Benutzers durch Fotografieren des Körpers eines Benutzers erfasst; und
die Behandlungsstellen-Erfassungseinheit so konfiguriert ist, dass sie die Behandlungsstelle aus dem Bild erfasst und ein Behandlungsstellensignal, das Informationen über die Behandlungsstelle enthält, an die Steuereinheit überträgt.

3. Lichttherapievorrichtung nach Anspruch 2, ferner umfassend:
eine zweite Bewegungseinheit zum Bewegen des Körpers der Behandlungseinheit in einer horizontalen Richtung.

4. Lichttherapievorrichtung nach Anspruch 3, wobei die Behandlungsstellenerkennungseinheit so konfiguriert ist, dass sie eine Stelle der Behandlungsstelle aus dem Bild erkennt und das Behandlungsstellensignal außerdem Informationen über die Stelle der Behandlungsstelle enthält.

5. Lichttherapievorrichtung nach Anspruch 4, wobei die Steuereinheit so konfiguriert ist, dass sie die zweite Bewegungseinheit so steuert, dass die Behandlungseinheit als Reaktion auf das Behandlungsstellensignal über der Behandlungsstelle positioniert wird.

6. Lichttherapievorrichtung nach Anspruch 1, wobei die Einheit zur Erkennung der Behandlungsstelle ferner umfasst:
eine erste Behandlungsstellen-Erfassungseinheit zum Erfassen eines Bildes des Körpers eines Benutzers durch Fotografieren des Körpers eines Benutzers und zum Erfassen einer Stelle der Behandlungsstelle anhand des Bildes; und
eine zweite Behandlungsstellen-Erfassungseinheit zum Erfassen der Behandlungsstelle durch Empfangen von Licht, das von einem Körper des Benutzers reflektiert wird.

7. Lichttherapievorrichtung nach Anspruch 6, ferner umfassend:
eine zweite Bewegungseinheit zum Bewegen des Körpers der Behandlungseinheit in einer horizontalen Richtung.

8. Lichttherapievorrichtung nach Anspruch 7, wobei die Steuerung so konfiguriert ist, dass sie die zweite Bewegungseinheit so steuert, dass die Behandlungseinheit als Reaktion auf das Signal der Behandlungsstelle über der Behandlungsstelle positioniert wird.

9. Lichttherapievorrichtung nach Anspruch 6, wobei die zweite Behandlungsstellen-Erfassungseinheit in der Lichtquelleneinheit angeordnet ist.

10. Lichttherapievorrichtung nach Anspruch 1, wobei eine Lichtquelle ein Substrat und einen auf dem Substrat angeordneten lichtemittierenden Chip umfasst.

11. Lichttherapievorrichtung nach Anspruch 10, wobei die Lichtquelle ferner eine auf dem Substrat angeordnete Körpererfassungseinheit umfasst.

12. Lichttherapievorrichtung nach Anspruch 11, wobei eine obere Fläche der Körpererfassungseinheit mit einer oberen Fläche der Lichtquelle bündig ist oder höher liegt als diese.

13. Lichttherapievorrichtung nach Anspruch 11, wobei die Steuerung so konfiguriert ist, dass sie den Betrieb der ersten Bewegungseinheit als Reaktion auf ein Körpererfassungssignal von der Körpererfassungseinheit stoppt.

14. Lichttherapievorrichtung nach Anspruch 13, wobei die Steuerung so konfiguriert ist, dass sie die Lichtquelleneinheit so steuert, dass sie das therapeutische Licht an die Behandlungsstelle abgibt, wenn sie das Körperdetektionssignal empfängt.

15. Lichttherapievorrichtung nach Anspruch 1, ferner umfassend:
einen Temperatursensor zum Erfassen einer Temperatur an oder um die Behandlungsstelle herum und zum Übertragen eines Temperatursignals an die Steuerung, wenn die erfasste Temperatur höher oder gleich einem vorbestimmten Wert ist.

## Revendications

1. Appareil de photothérapie comprenant :
une unité de détection du site de traitement pour détecter un site de traitement dans le corps d'un utilisateur ;
une unité de traitement comprenant une première unité mobile dans une direction verticale, un corps monté sur la première unité mobile, une unité de source de lumière comprenant de multiples sources de lumière disposées sur une surface inférieure du corps et émettant une lumière thérapeutique ; et
un contrôleur pour commander le fonctionnement de la première unité mobile et de l'unité de source lumineuse, le contrôleur étant configuré pour commander :
la détection du site de traitement par l'unité de détection du site de traitement, la première unité mobile pour amener l'unité de source lumineuse en contact avec le site de traitement ; et,
sur la détermination du contact entre l'unité de source lumineuse et le site de traitement, l'unité de source lumineuse positionnée sur le site de traitement pour émettre la lumière thérapeutique ; et,
le corps de l'unité de traitement est déformable sous l'effet de la pression exercée par le site de traitement sur les multiples sources de lumière et, lorsque la pression est relâchée, le corps de l'unité de traitement est configuré pour reprendre sa forme initiale.

2. Appareil de photothérapie selon la revendication 1, dans lequel :
l'unité de détection du site de traitement est configurée pour acquérir une image du corps de l'utilisateur par le biais d'une photographie du corps de l'utilisateur ; et
l'unité de détection du site de traitement est configurée pour détecter le site de traitement à partir de l'image et pour transmettre au contrôleur un signal de site de traitement contenant des informations sur le site de traitement.

3. Appareil de photothérapie selon la revendication 2, comprenant en outre :
une deuxième unité de déplacement pour déplacer le corps de l'unité de traitement dans une direction horizontale.

4. Appareil de photothérapie selon la revendication 3, dans lequel l'unité de détection du site de traitement est configurée pour détecter un emplacement du site de traitement à partir de l'image et le signal du site de traitement contient en outre des informations sur l'emplacement du site de traitement.

5. Appareil de photothérapie selon la revendication 4, dans lequel le contrôleur est configuré pour commander la deuxième unité mobile de sorte que l'unité de traitement soit positionnée sur le site de traitement en réponse au signal de site de traitement.

6. Appareil de photothérapie selon la revendication 1, dans lequel l'unité de détection du site de traitement comprend en outre :
une première unité de détection du site de traitement pour acquérir une image du corps de l'utilisateur par photographie du corps de l'utilisateur et détecter l'emplacement du site de traitement à partir de l'image ; et
une deuxième unité de détection du site de traitement pour détecter le site de traitement en recevant la lumière réfléchie par le corps de l'utilisateur.

7. Appareil de photothérapie selon la revendication 6, comprenant en outre :
une deuxième unité de déplacement pour déplacer le corps de l'unité de traitement dans une direction horizontale.

8. Appareil de photothérapie selon la revendication 7, dans lequel le contrôleur est configuré pour commander la deuxième unité de déplacement de sorte que l'unité de traitement soit positionnée sur le site de traitement en réponse au signal du site de traitement.

9. Appareil de photothérapie selon la revendication 6, dans lequel la deuxième unité de détection du site de traitement est disposée dans l'unité de source lumineuse.

10. Appareil de photothérapie selon la revendication 1, dans lequel une source de lumière comprend un substrat et une puce émettrice de lumière disposée sur le substrat.

11. Appareil de photothérapie selon la revendication 10, dans lequel la source de lumière comprend en outre une unité de détection du corps disposée sur le substrat.

12. Appareil de photothérapie selon la revendication 11, dans lequel une surface supérieure de l'unité de détection du corps est au même niveau qu'une surface supérieure de la source de lumière ou plus haute que celle-ci.

13. Appareil de photothérapie selon la revendication 11, dans lequel le contrôleur est configuré pour arrêter le fonctionnement de la première unité mobile en réponse à un signal de détection du corps provenant de l'unité de détection du corps.

14. Appareil de photothérapie selon la revendication 13, dans lequel le contrôleur est configuré pour commander l'unité de source lumineuse afin de délivrer la lumière thérapeutique au site de traitement lors de la réception du signal de détection du corps.

15. Appareil de photothérapie selon la revendication 1, comprenant en outre :
un capteur de température pour détecter une température au niveau ou autour du site de traitement et pour transmettre un signal de température au contrôleur lorsque la température détectée est supérieure ou égale à une valeur prédéterminée.
